Europäisches Patentamt

European Patent Office

Office européen des brevets

(11) Veröffentlichungsnummer: **0 185 865**
**A1**

(12) # EUROPÄISCHE PATENTANMELDUNG

(21) Anmeldenummer: 85112849.6

(22) Anmeldetag: 10.10.85

(51) Int. Cl.⁴: **A 61 M 25/00**
**B 32 B 1/08**

(30) Priorität: 18.12.84 DE 3446033

(43) Veröffentlichungstag der Anmeldung:
02.07.86 Patentblatt 86/27

(84) Benannte Vertragsstaaten:
AT BE CH DE FR GB IT LI LU NL SE

(71) Anmelder: INTERMEDICAT GMBH
Gerliswilstrasse 74
CH-6020 Emmenbrücke(CH)

(72) Erfinder: Aigner, Karl, Dr.
Klinikstrasse 29
D-6300 Giessen(DE)

(72) Erfinder: Löffler, Thomas, Dr.
Breurhausstrasse 40
D-4600 Dortmund(DE)

(72) Erfinder: Stöber, Herbert, Dr.
Über dem Rötter 1
D-3513 Staufenberg 1(DE)

(74) Vertreter: von Kreisler, Alek, Dipl.-Chem. et al,
Deichmannhaus am Hauptbahnhof
D-5000 Köln 1(DE)

(54) Implantierbarer intraperitonealer Katheter.

(57) Am distalen Endbereich des Katheterschlauches (10) sind seitliche Löcher (11) in Längsrichtung hintereinander vorgesehen. Zwischen den Löchern (11) befinden sich scheibenförmige Distanzelemente (13), die radial von dem Katheterschlauch abstehen und verhindern, daß die Löcher (11) durch Gewebeüberwucherungen verschlossen werden.

FIG.1

Croydon Printing Company Ltd.

Implantierbarer intraperitonealer Katheter

Die Erfindung betrifft einen implantierbaren intraperitonealen Katheter zum Zuführen von Wirkstoffen in den
Körper, mit einem Katheterschlauch, der am distalen
Endbereich seitliche Löcher mit axialen Abständen aufweist.

Die Notwendigkeit, bestimmte Wirkstoffe, z.B. Zytostatika, intraperitoneal (d.h. in die Bauchhöhle) zuzuführen, ist in den letzten Jahren in zunehmendem Maße erkannt worden. Es handelt sich hierbei meist um länger
dauernde bzw. auch lebenslange Therapien, die eine sichere Applikation über ein implantierbares System über
lange Zeiträume erforderlich machen.

Bekannt sind intraperitoneale Katheter aus einem Katheterschlauch, der am distalen Ende seitliche Löcher mit
axialen Abständen aufweist. Es hat sich gezeigt, daß
die Löcher in vielen Fällen schon nach kurzer Zeit durch Proliferation (Wucherung) des umgebenden Gewebes überdeckt und verschlossen werden können, so daß die zuzuführenden Wirkstoffe den Katheter nicht oder nicht in hinreichendem Maße verlassen können.

Der Erfindung liegt die Aufgabe zugrunde, einen Katheter der eingangs genannten Art zu schaffen, bei dem sichergestellt ist, daß die Löcher am distalen Katheterende nicht überwuchert werden können.

Die Lösung dieser Aufgabe besteht erfindungsgemäß darin, daß zwischen den Löchern axial hintereinander angeordnete seitlich abstehende Distanzelemente angeordnet sind, deren gegenseitige Abstände so bemessen sind, daß das Vordringen von Gewebewucherungen bis an die Löcher verhindert wird.

Die Distanzelemente verhindern, daß Gewebeproliferationen die Löcher überdecken und verschließen. Die Löcher sind jeweils zwischen zwei Distanzelementen versenkt untergebracht und können von Gewebewucherungen nicht erreicht werden.

Der erfindungsgemäße Katheter eignet sich beispielsweise für die intraperitoneale Zufuhr von Zytostatika bei Ovarial- und Peritoneal-Karzinomen sowie für die Insulin-Zufuhr bei Diabetikern mit peripherer Insulin-Resistenz.

Die Distanzelemente sind vorzugsweise als radiale Scheiben ausgebildet. Diese Scheiben begrenzen ringförmige Räume, die den Katheterschlauch umgeben und aus denen der Wirkstoff auf dem gesamten Umfang, der größer ist als derjenige des Katheterschlauchs, austreten kann. Die Gefahr von Überwucherungen durch das Bindege-

webe ist hierbei wegen der relativ großen Durchmesser der Distanzelemente nahezu vollständig ausgeschlossen. Andererseits brauchen die Distanzelemente nicht notwendigerweise als ringförmige Scheiben ausgebildet zu sein. Es mag in einigen Fällen ausreichen, nur denjenigen Umfangsbereich des Katheterschlauchs mit Distanzelementen zu versehen, in dem sich die Löcher befinden.

Zweckmäßigerweise sind die Distanzelemente dem Katheterschlauch einstückig angeformt.

Ein weiteres Problem bei implantierbaren Kathetern besteht darin, daß diese Katheter entweder mit dem zu applizierenden Wirkstoff oder mit dem Körpergewebe unverträglich sind. Zur Überwindung dieser Schwierigkeit schlägt die Erfindung vor, daß der Katheterschlauch an seiner Innenfläche ein Material aufweist, das inert gegenüber dem zuzuführenden Wirkstoff ist und an seiner Außenfläche sowie an den Distanzelementen ein anderes Material, das biokompatibel (d.h. verträglich mit Gewebe und/oder Blut) ist.

Der Radius und die axialen Abstände der Distanzelemente sollten so gewählt sein, daß Gewebewucherungen daran gehindert werden, in die Räume zwischen den Distanzelementen einzudringen.

Im folgenden wird unter Bezugnahme auf die Zeichnungen ein Ausführungsbeispiel der Erfindung näher erläutert.

Es zeigen:

    Fig. 1 eine Seitenansicht des distalen Endbereichs des Katheters und

    Fig. 2 einen Schnitt entlang der Linie II-II nach Fig. 1 in vergrößertem Maßstab.

Der dargestellte implantierbare intraperitoneale Katheter weist einen rohrförmigen flexiblen Katheterschlauch 10 auf, der am proximalen Ende mit einem (nicht dargestellten) Zuspritzteil versehen ist, das ebenfalls implantierbar ist oder mit einem Konnektor für den Anschluß an eine extrakorporale oder implantierbare Pumpe. Am distalen Ende sind mehrere in axialer Richtung des Katheterschlauches hintereinander angeordnete Löcher 11 vorgesehen, aus denen die zu applizierende Flüssigkeit seitlich aus dem Katheterschlauch austreten kann. Das Ende 12 des Katheterschlauchs ist bei dem vorliegenden Ausführungsbeispiel verschlossen, könnte jedoch auch als zusätzliche Öffnung ausgebildet sein.

Zwischen zwei Löchern 11, die in axialer Richtung des Katheterschlauches 10 mit Abständen hintereinander angeordnet sind, befindet sich jeweils ein Distanzelement 13 in Form einer seitlich abstehenden radialen Scheibe, die dem Katheterschlauch 10 einstückig angeformt ist. Die Außendurchmesser der Distanzelemente 13 betragen mindestens das Dreifache, vorzugsweise mindestens das Vierfache, des Außendurchmessers des Katheterschlauches. Der Katheterschlauch und die Distanzelemente 13 bestehen aus weichem flexiblem Material. Die Abstände der Distanzelemente 13 sind gleich den Abständen der Löcher 11. Diese Abstände sind nur geringfügig größer als der Lochdurchmesser. Vorzugsweise sind die Abstände der Distanzelemente 13 von den Löchern 11 kleiner als der Lochdurchmesser.

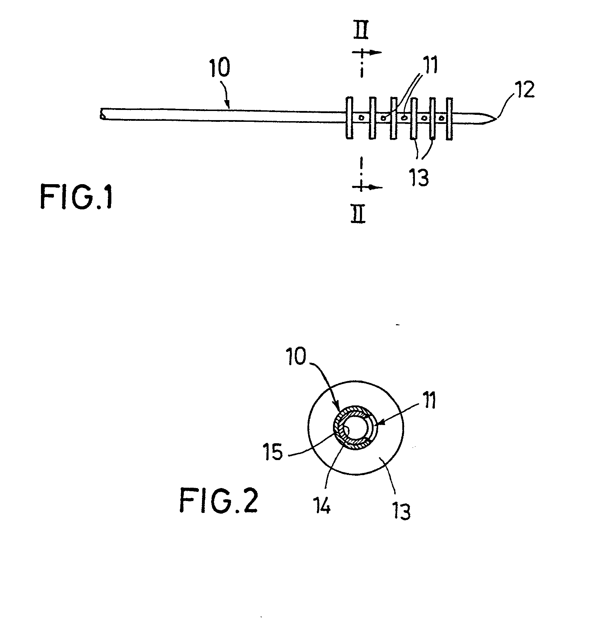

Wie Fig. 2 zeigt, besteht der Katheterschlauch 10 aus zwei Schichten, von denen die äußere Schicht 14 aus biokompatiblem Kunststoff besteht, während die innere Schicht 15 aus einem gegenüber dem zu gebenden Wirkstoff inerten Kunststoff besteht. Die Löcher 11, die beispielsweise durch Ausstanzung erzeugt worden sind, erstrecken sich durch beide Schichten 14, 15 hindurch.

Die Verlegung des Katheters erfolgt operativ. Alternativ ist es auch möglich, den Katheter mit bekannten Einführtechniken subkutan zu verlegen.

Bei bestehender peripherer Insulinresistenz ist es erforderlich, das Insulin intraperitoneal zu verabfolgen. Von flexiblen, zur Herstellung von Kathetern geeigneten polymeren Werkstoffen wie thermoplastischen Polyurethanen oder elastomerem Silikongummi oder weichgemachtem Polyvinylchlorid ist bekannt, daß sie Insulin und andere Wirkstoffe (z.B. Nitroglycerin) in beachtlicher Menge adsorbieren bzw. auch durch das Material penetrieren lassen. Deshalb wird gefordert, daß die Innenseite des Katheters aus einem Material besteht, das nicht zu Interaktionen mit Wirkstoffen neigt, z.B. PE. Die Außenseite sollte dagegen biokompatibel gegenüber Blut und/oder Gewebe sein.

Zweischichtige, den zwei- (und mehrschichtigen) Verbundfolien aus thermoplastischen Werkstoffen analog aufgebaute Katheterschläuche können im Coextrusionsverfahren aus konzentrischen Ringspaltdüsen austretendem plastifiziertem Kunststoff geformt werden und haben eine innere kontinuierliche Schicht aus nicht oder wenig sorbierendem Polymerwerkstoff und eine äußere kontinuierliche Schicht, die die mechanischen Eigenschaften überwiegend bestimmt.

Geeignete Werkstoffkombinationen sind z.B. Polyolefine wie Polyethylen oder Polypropylen als innere, nicht oder nur wenig sorbierende Schicht in einer Dicke von 50 - 100 µm, kombiniert mit einem thermoplastischen, biokompatiblen Polyurethan oder segmentiertem Polyestercopolymeren (Hytrel $^R$, DuPont) oder Polyetheresteramid als äußere Schicht mit einer Dicke von 0,4-0,5 mm, so daß sich ein Katheterschlauch mit einer Gesamtwanddicke von 0,45-0,6 mm ergibt. Es liegt im Bereich der Erfindung, daß auch andere Wanddicken und ein daraus resultierendes anderes Wanddickenverhältnis gewählt werden können.

ANSPRÜCHE

1. Implantierbarer intraperitonealer Katheter zum Zuführen von Wirkstoffen in den Körper, mit einem Katheterschlauch, der am distalen Endbereich seitliche Löcher mit axialen Abständen aufweist, dadurch gekennzeichnet, daß zwischen den Löchern (11) axial hintereinander angeordnete seitlich abstehende Distanzelemente (13) angeordnet sind, deren gegenseitige Abstände so bemessen sind, daß das Vordringen von Gewebewucherungen bis an die Löcher (11) verhindert wird.

2. Katheter nach Anspruch 1, dadurch gekennzeichnet, daß die Distanzelemente (13) radiale Scheiben sind.

3. Katheter nach Anspruch 1 oder 2, dadurch gekennzeichnet, daß die Distanzelemente (13) dem Katheterschlauch (10) einstückig angeformt sind.

4. Katheter nach einem der Ansprüche 1 bis 3, dadurch gekennzeichnet, daß der Katheterschlauch (10) an seiner Innenfläche ein Material (14) aufweist, das inert gegenüber dem zuzuführenden Wirkstoff ist, und an seiner Außenfläche sowie an den Distanzelementen (13) ein anderes Material (15), das biokompatibel ist.

5. Katheter nach einem der Ansprüche 1 bis 4, dadurch gekennzeichnet, daß der Radius der Distanzelemente (13) mindestens dreimal so groß ist wie derjenige des Katheterschlauches (10).

6. Katheter nach einem der Ansprüche 1 bis 5, dadurch gekennzeichnet, daß die Distanzelemente (13) einen axialen Abstand voneinander haben, der gleich dem axialen Abstand der Löcher (11) ist.

7. Katheter nach einem der Ansprüche 1 bis 6, dadurch gekennzeichnet, daß die Abstände der Distanzelemente (13) von den Löchern (11) nicht wesentlich größer sind als der Lochdurchmesser.

8. Katheter nach einem der Ansprüche 1 bis 7, dadurch gekennzeichnet, daß die Löcher radial versetzt zwischen den Distanzelementen angeordnet sind.

FIG.1

FIG.2

Europäisches
Patentamt

**EUROPÄISCHER RECHERCHENBERICHT**

**0185865**
Nummer der Anmeldung

EP 85 11 2849

## EINSCHLÄGIGE DOKUMENTE

| Kategorie | Kennzeichnung des Dokuments mit Angabe soweit erforderlich, der maßgeblichen Teile | Betrifft Anspruch | KLASSIFIKATION DER ANMELDUNG (Int Cl 4) |
|---|---|---|---|
| X | DE-A-1 900 227 (HAKIM)<br><br>* Insgesamt * | 1-3,5-8 | A 61 M 25/00<br>B 32 B 1/08 |
| Y | | 4 | |
| | --- | | |
| Y | FR-A-2 334 042 (KABEL- UND METALLWERKE GUTEHOFFNUNGSHUTTE AG)<br>* Figur 1; Seite 5, Zeilen 23-37 * | 4 | |
| | --- | | |
| X | US-A-3 894 541 (EL-SHAFEI)<br>* Figur 2; Spalte 3, Zeilen 3-29 * | 1-3 | |
| | --- | | RECHERCHIERTE SACHGEBIETE (Int Cl 4) |
| A | FR-A-2 320 184 (ALLGEMEINE SYNTHETISCHE GESELLSCHAFT) | | |
| | ----- | | A 61 M<br>B 32 B |

Der vorliegende Recherchenbericht wurde für alle Patentansprüche erstellt

| Recherchenort<br>DEN HAAG | Abschlußdatum der Recherche<br>26-03-1986 | Prüfer<br>DEUTSCH J.P.M. |
|---|---|---|

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82